(19) Europäisches Patentamt
European Patent Office
Office européen des brevets

(11) Veröffentlichungsnummer: **0 100 476**
**A2**

(12) # EUROPÄISCHE PATENTANMELDUNG

(21) Anmeldenummer: 83106979.4

(22) Anmeldetag: 16.07.83

(51) Int. Cl.³: C 07 F 9/65
A 01 N 57/16, A 01 N 57/24
A 01 N 57/32

(30) Priorität: 29.07.82 DE 3228340

(43) Veröffentlichungstag der Anmeldung: 15.02.84 Patentblatt 84/7

(84) Benannte Vertragsstaaten: AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Maurer, Fritz, Dr.
Roeberstrasse 8
D-5600 Wuppertal 1(DE)

(72) Erfinder: Hammann, Ingeborg, Dr.
Lutherstrasse 22
D-4330 Mülheim/Ruhr(DE)

(72) Erfinder: Homeyer, Bernhard, Dr.
Obere Strasse 28
D-5090 Leverkusen 3(DE)

(54) **Phosphorsäureester, Verfahren zu ihrer Herstellung und ihre Verwendung als Schädlingsbekämpfungsmittel.**

(57) Die Erfindung betrifft neue Phosphorsäureester der Formel I

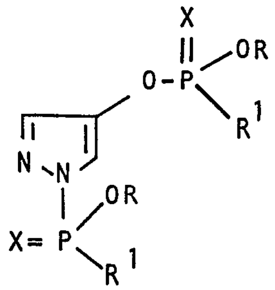

(I)

in welcher
R für gegebenenfalls substituiertes Alkyl steht,
$R^1$ für gegebenenfalls substituierte Reste aus der Reihe Alkyl, Alkoxy, Alkylthio, (Di)Alkylamino oder Aryl steht, und
X für Sauerstoff oder Schwefel steht, welche als Schädlingsbekämpfungsmittel verwendet werden können.

EP 0 100 476 A2

BAYER AKTIENGESELLSCHAFT 5090 Leverkusen, Bayerwerk

Zentralbereich
Patente, Marken und Lizenzen S/AB
Ia

# Phosphorsäureester, Verfahren zu ihrer Herstellung und ihre Verwendung als Schädlingsbekämpfungsmittel

Die Erfindung betrifft neue Phosphorsäureester, ein Verfahren zu ihrer Herstellung und ihre Verwendung als Schädlingsbekämpfungsmittel, insbesondere als Insektizide.

Es ist bekannt, daß bestimmte O-Pyrazolyl-phosphorsäureester wie z.B. O-Ethyl-S-n-propyl-O-(1-methyl-4-methylthio-5-pyrazolyl)-dithiophosphorsäureester, O-Ethyl-S-n-propyl-O-[3-methyl-1-(ethoxy-n-propylthio-thionophosphoryl)-5-pyrazolyl]-dithiophosphorsäureester, O,O-Diethyl-O-[3-methyl-1-(diethoxy-thionophosphoryl)-5-pyrazolyl]-thionophosphorsäureester und O,O-Diethyl-O-(3-methyl-1-phenyl-5-pyrazolyl)-thionophosphorsäureester, insektizide Eigenschaften aufweisen (vergleiche DE-OS 2 603 215, 2 548 183 DE-OS 1 917 741 und AT-PS 184 580). Die insektizide Wirkung dieser bekannten Verbindungen ist jedoch unter bestimmten Umständen, insbesondere bei niedrigen Wirkstoffkonzentrationen und Aufwandmengen, nicht immer voll zufriedenstellend.

Es wurden nun neue O-(1-Phosphoryl-4-pyrazolyl)-phosphorsäureester der Formel I

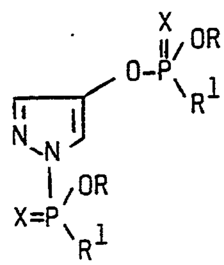

(I)

gefunden, in welcher

R für gegebenenfalls substituiertes Alkyl steht,

$R^1$ für gegebenenfalls substituierte Reste aus der Reihe Alkyl, Alkoxy, Alkylthio, (Di)Alkylamino oder Aryl steht, und

X für Sauerstoff oder Schwefel steht.

Man erhält die neuen Phosphorsäureester der Formel I, wenn man 4-Hydroxypyrazol der Formel II

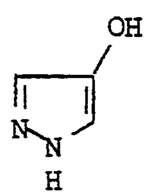

(II)

oder deren Alkalimetall-, Erdalkalimetall- oder Ammoniumsalze mit Halogeniden der Formel III

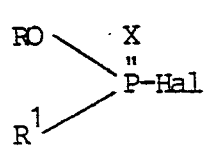

(III)

in welcher

R, $R^1$ und X die oben angegebenen Bedeutungen haben, und

Hal für Halogen (vorzugsweise Chlor oder Brom) steht,

gegebenenfalls in Gegenwart eines Säureakzeptors und gegegebenfalls unter Verwendung eines Verdünnungsmittels umsetzt.

Die neuen O-(1-Phosphoryl-4-pyrazolyl)-phosphorsäureester der Formel (I) zeichnen sich durch hohe Wirksamkeit als Schädlingsbekämpfungsmittel, insbesondere durch ihre hervorragende insektizide Wirkung aus.

Die gegebenenfalls substituierten Alkylreste R und $R^1$ sowie die Alkylteile der gegebenenfalls substituierten Alkoxy-, Alkylthio-, Alkylamino- und Dialkylaminoreste $R^1$ können verzweigt oder unverzweigt sein und enthalten vorzugsweise jeweils 1 bis 8, insbesondere 1 bis 6 und besonders bevorzugt 1 bis 4 Kohlenstoffatome.

Besonders bevorzugt stehen R für gegebenenfalls substituiertes Methyl, Ethyl, n-Propyl, iso-Propyl, n-Butyl, iso-Butyl und tert.-Butyl; $R^1$ für gegebenenfalls substituiertes Methyl, Ethyl, n-Propyl, iso-Propyl, n-Butyl, iso-Butyl, tert.-Butyl, Methoxy, Ethoxy, n-Propoxy, iso-Propoxy, n-Butoxy, sec.-Butoxy, iso-Butoxy, tert.-Butoxy, Methylthio, Ethylthio, n-Propylthio, iso-Propylthio, n-Butylthio, iso-Butylthio, sec.-Butylthio, tert-Butylthio, (Di)-Methyl-amino, (Di)Ethylamino, (Di)n-Propylamino, (Di)-isopropylaminmo, (Di)-n-Butylamino, (Di)-iso-Butylamino, (Di)-sec.-Butylamino und (Di)-tert-Butylamino oder Phenyl.

Die gegebenenfalls substituierten Reste in der Definition von R und $R^1$ können einen oder mehrere, vorzugsweise 1 bis 3, insbesondere 1 oder 2 gleiche oder verschiedene Substitutenten tragen. Beispielsweise seien genannt: Halogene, wie Fluor, Chlor und Brom, Nitro, Cyano sowie Alkyl und Alkylthio mit vorzugsweise 1 bis 5, insbesondere 1 bis 3 Kohlenstoffatomen.

Bevorzugt sind die Reste R und $R^1$ jedoch unsubstituiert.

Die Erfindung betrifft vorzugsweise Verbindungen der Formel (I), in welcher

R für geradkettiges oder verzweigtes Alkyl mit 1 bis 6 Kohlenstoffatomen steht,

$R^1$ für Alkyl, Alkoxy, Alkylthio und (Di)Alkylamino, jeweils mit geradkettigem oder verzweigtem Alkylrest und 1 bis 6 Kohlenstoffatomen, oder für Phenyl steht, und

X für Sauerstoff oder Schwefel steht.

Ganz besonders bevorzugt sind die Verbindungen der Formel (I), in welcher

R für Methyl, Ethyl, n-Propyl, i-Propyl ,n-Butyl, i-Butyl, sec.-Butyl und tert.-Butyl steht,

$R^1$ für Methyl, Ethyl, n-Propyl, i-Propyl, n-Butyl, i-Butyl, Methoxy, Ethoxy, n-Propoxy, i-Propoxy, n-Butoxy, i-Butoxy, Methylthio, Ethylthio, n-Propylthio, i-Propylthio, n-Butylthio, i-Butylthio, sec.-Butylthio, (Di)Methylamino, (Di)Ethylamino, (Di)n-Propylamino, (Di)i-Propylamino, (Di)n-Butylamino,

(Di)iso-Butylamino, Methylethylamino, Methyl-n-propylamino, Methyl-i-propylamino, Methyl-n-butylamino, Methyl-i-butylamino, Ethyl-n-propylamino, Ethyl-i-propylamino, Ethyl-n-butylamino, Ethyl-i-butylamino, n-Propyl-i-propylamino, n-Propyl-n-butylamino, n-Propyl-i-butylamino, i-Propyl-n-butylamino, i-Propyl-i-butylamino, n-Butyl-i-butylamino und Phenyl steht, und

X für Sauerstoff oder Schwefel steht.

Verwendet man beispielsweise für das erfindungsgemäße Verfahren als Ausgangsstoffe 4-Hydroxypyrazol oder das entsprechende Natriumsalz und O,O-Diethyl-phosphorsäureesterchlorid, so können die entsprechenden Reaktionen durch folgende Formelschemata skizziert werden :

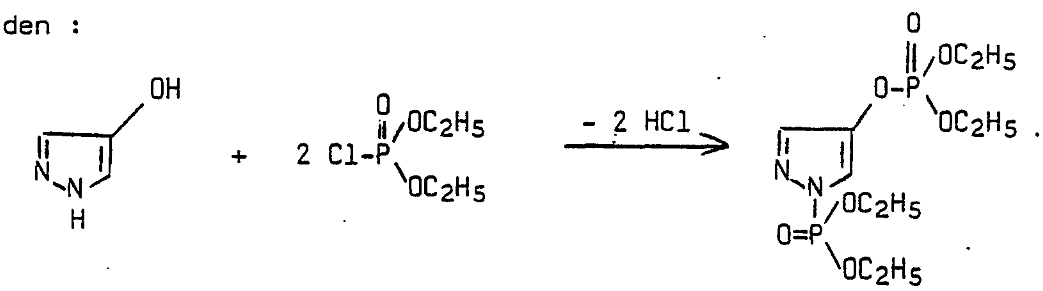


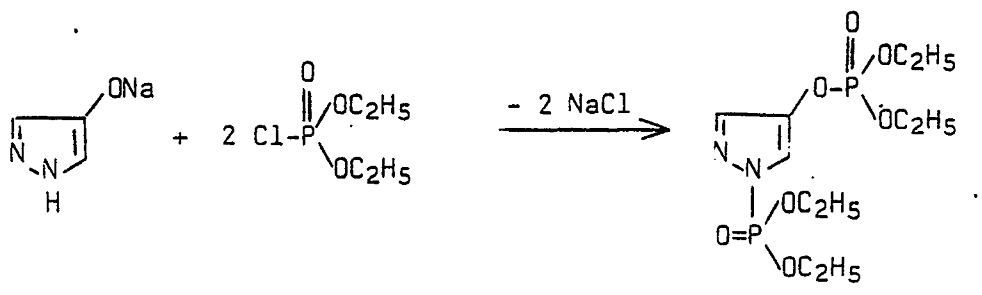


Das beim erfindungsgemäßen Verfahren als Ausgangsstoff zu verwendende 4-Hydroxy-pyrazol bzw.dessen Salze sind durch die Formel II

definiert. Die Alkali- und Erdalkalimetallionen stehen vorzugsweise für Natrium-, Kalium-, Calcium- und Ammoniumionen.

Die 4-Hydroxy-pyrazol bzw. die entsprechenden Ammonium- oder Alkali- und Erdalkalimetall-salze können nach allgemein bekannten Verfahren und Methoden hergestellt werden (vgl. z.B. Liebigs Ann. Chem. 313, (1900), 17 und DE-OS 2 931 033).

Die ebenfalls als Ausgangsstoffe für die Herstellung der neuen Verbindungen der Formel (I) einzusetzenden Halogenide sind durch die Formel (III) definiert.

Als Beispiele für die Ausgangsstoffe der Formel (III) seien genannt :

Methoxy-methyl-, Methoxy-ethyl-, Methoxy-n-propyl-, Methoxy-i-propyl-, Methoxy-n-butyl-, Methoxy-i-butyl-, Ethoxy-methyl-, Ethoxy-ethyl-, Ethoxy-n-propyl-, Ethoxy-i-propyl-, Ethoxy-n-butyl-, Ethoxy-i-butyl-, n-Propoxy-methyl-, n-Propoxy-ethyl-, n-Propoxy-n-propyl-, n-Propoxy-i-propyl-, n-Propoxy-n-butyl-, n-Propoxy-i-butyl-, i-Propoxy-methyl-, i-Propoxy-ethyl-, i-Propoxy-n-propyl-, i-Propoxy-i-propyl-, i-Propoxy-n-butyl-, i-Propoxy-i-butyl-, n-Butoxy-methyl-, n-Butoxy-ethyl-, n-Butoxy-n-propyl-, n-Butoxy-i-propyl-, n-Butoxy-n-butyl-, n-Butoxy-i-butyl-, i-Butoxy-methyl-, i-Butoxy-ethyl-, i-Butoxy-n-propyl-, i-Butoxy-i-propyl-, i-Butoxy-n-butyl-, i-Butoxy-i-butyl-, sec.-Butoxy-methyl-, sec.-Butoxy-ethyl-, sec.-Butoxy-n-propyl-, sec.-Butoxy-i-propyl-, sec.-Butoxy-n-butyl-, sec.-Butoxy-i-butyl-, tert.-Butoxy-methyl-, tert.-Butoxy-ethyl-, tert.-Butoxy-n-propyl-, tert.-Butoxy-i-propyl-, tert.-Butoxy-n-butyl-, tert.-Butoxy-i-butyl-(thiono)phosphonsäureester-chlorid bzw. -bromid;

Dimethoxy-, Diethoxy-, Di-n-propoxy-, Di-i-propoxy-, Di-n-butoxy-, Di-i-butoxy-, Methoxy-ethoxy-, Methoxy-n-propoxy-, Methoxy-i-propoxy-, Methoxy-n-butoxy-, Methoxy-i-butoxy-, Methoxy-sec.-butoxy-, Methoxy-tert.-butoxy-, Ethoxy-n-propoxy-, -, Ethoxy-i-propoxy-, Ethoxy-n-butoxy-, Ethoxy-i-butoxy-, Ethoxy-sec.-butoxy-, Ethoxy-tert.-butoxy-, n-Propoxy-i-propoxy-, n-Propoxy-n-butoxy-, n-Propoxy-i-butoxy-, n-Propoxy-sec.-butoxy-, n-Propoxy-tert.-butoxy-, i-Propoxy-n-butoxy-, i-Propoxy-i-butoxy-, n-Butoxy-i-butoxy-, n-Butoxy-sec.-butoxy-, n-Butoxy-tert.-butoxy-(thiono)phosphorsäureester-chlorid bzw. -bromid;

Methoxy-methylthio-, Methoxy-ethylthio-, Methoxy-n-propylthio-, Methoxy-i-propylthio-, Methoxy-n-butylthio-, Methoxy-i-butylthio-, Ethoxy-methylthio-, Ethoxy-ethylthio-, Ethoxy-n-propylthio-, Ethoxy-i-propylthio-, Ethoxy-n-butylthio-, Ethoxy-i-butylthio-, Ethoxy-sec.-butylthio-, n-Propoxy-methylthio-, n-Propoxy-ethylthio-, n-Propoxy-n-propylthio-, n-Propoxy-i-propylthio-, n-Propoxy-n-butylthio-, n-Propoxy-i-butylthio-, i-Propoxy-methylthio-, i-Propoxy-ethylthio-, i-Propoxy-n-propylthio-, i-Propoxy-i-propylthio-, i-Propoxy-n-butylthio-, i-Propoxy-i-butylthio-, n-Butoxy-methylthio-, n-Butoxy-ethylthio-, n-Butoxy-n-propylthio-, n-Butoxy-i-propylthio-, n-Butoxy-n-butylthio-, n-Butoxy-i-butylthio-, i-Butoxy-methylthio-, i-Butoxy-ethylthio-, i-Butoxy-n-propylthio-, i-Butoxy-i-propylthio-, i-Butoxy-n-butylthio-, i-Butoxy-i-butylthio-(thiono)phosphorsäureester-chlorid bzw. -bromid;

Methoxy-(di)methylamino-, Methoxy-(di)ethylamino-, Methoxy-(di)-n-propylamino-, Methoxy-(di)i-propylamino-, Methoxy-(di)n-butylamino-, Methoxy-(di)i-butylamino-, Ethoxy-(di)methylamino-, Ethoxy-(di)n-propylamino-, Ethoxy-(di)i-propylamino-, Ethoxy-(di)-n-butylamino-, Ethoxy-(di)i-butylamino-, n-Propoxy-(di)methylamino-, n-Propoxy-(di)ethylamino-, n-Propoxy-(di)n-propylamino-,

n-Propoxy-(di)i-propylamino-, n-Propoxy-(di)n-butylamino-, n-Propoxy-(di)i-butylamino-, i-Propoxy-(di)methylamino-, i-Propoxy-(di)ethylamino-, i-Propoxy-(di)n-propylamino-, i-Propoxy-(di)i-propylamino-, i-Propoxy-(di)n-butylamino-, i-Propoxy-(di)i-butylamino-, n-Butoxy-(di)methylamino-, n-Butoxy-(di)ethylamino-, n-Butoxy-(di)-n-propylamino-, n-Butoxy-(di)i-propylamino-, n-Butoxy-(di)n-butylamino-, n-Butoxy-(di)i-butylamino-, i-Butoxy-(di)methylamino-, i-Butoxy-(di)ethylamino-, i-Butoxy-(di)n-propylamino-, i-Butoxy-(di)i-propylamino-, i-Butoxy-(di)n-butylamino-, i-Butoxy-(di)i-butylamino-, sec.-Butoxy-(di)methylamino-, sec.-Butoxy-(di)ethylamino-, sec.-Butoxy-(di)n-propylamino-, sec.-Butoxy-(di)i-propylamino-, sec.-Butoxy-(di)i-butylamino-, tert.-Butoxy-(di)methylamino-, tert.-Butoxy-(di)ethylamino-, tert.-Butoxy-(di)n-propylamino-, tert.-Butoxy-(di)n-butylamino-(thiono)phosphorsäureester-chlorid bzw. -bromid;

Methoxy-, Ethoxy-, n-Propoxy-, i-Propoxy-, n-Butoxy-, i-Butoxy-, sec.-Butoxy- und tert.-Butoxy-phenyl(thiono)phosphonsäure-chlorid bzw. -bromid.

Die Verbindungen der Formel (III) sind bekannt und/oder können nach allgemein bekannten Verfahren und Methoden hergestellt werden (vgl. z.B. Methoden der organischen Chemie (Houben-Weyl-Müller), 4. Aufl., Band 12/1 (1963), S. 415-420 und S. 560-563; Band 12/2 (1964), S. 274-292, S. 405-408 und S. 607-618, S. 621-622 und S. 755-757; Thieme-Verlag Stuttgart).

Das erfindungsgemäße Verfahren zur Herstellung der neuen O-(1-Phosphoryl-4-pyrazolyl)-phosphorsäureester der Formel I wird bevorzugt unter Verwendung von Verdünnungsmitteln durchgeführt. Als Verdünnungsmittel kommen praktisch alle inerten organischen Lösungsmittel in Frage.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Das Verfahren kann gegebenenfalls in Gegenwart von Säureakzeptoren durchgeführt werden. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Das erfindungsgemäße Verfahren wird im allgemeinen bei Temperaturen zwischen 0 und 100°C durchgeführt. Bevorzugt wird der Bereich zwischen 20 und 80°C. Die Umsetzungen werden im allgemeinen bei Normaldruck durchgeführt.

Zur Durchführung des erfindungsgemäßen Verfahrens setzt man auf 1 Mol 4-Hydroxypyrazol bzw. dessen Ammonium-, Alkali- und Erdalkalimetallsalz 2 bis 3 Mol vorzugsweise 2 bis 2,2 Mol Halogenid der Formel (III) ein.
Die Umsetzung wird im allgemeinen in einem geeigneten Verdünnungsmittel und gegebenenfalls in Gegenwart eines Säureakzeptors durchgeführt. Die Aufarbeitung geschieht nach üblichen Methoden. Die neuen Verbindungen fallen zum Teil in Form von Oelen an, die sich zum Teil nicht unzersetzt destillieren lassen, jedoch durch sogenanntes 'Andestillieren', d.h. längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirkstoffe eignen sich bei guter Pflanzenverträglichkeit zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der

Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren

handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Laphygma-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigte z.B. bei einer Wirkstoffkonzentration von 0,01 % die Verbindung des Herstellungsbeispiel 1 nach 3 Tagen eine Abtötung von 100 %.

Beispiel B

Phaedon-Larven-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B. bei einer Wirkstoffkonzentration von 0,01 % die Verbindungen der Herstellungsbeispiele 3 und 8 nach 3 Tagen eine Abtötung von 100%.

Beispiel C

Doralis-Test (systemische Wirkung)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigten z.B. bei einer Wirkstoffkonzentration von 0,1 % die Verbindungen der Herstellungsbeispiele 2 und 5 nach 4 Tagen eine Abtötung von 100%.

Beispiel D

Tetranychus-Test (resistent)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigten z.B. bei einer Wirkstoffkonzentration von 0,1 % nach 2 Tagen die Verbindungen der Herstellungsbeispiele 1 und 2 eine 90 bis 100 % Abtötung.

Beispiel E

Grenzkonzentrations-Test / Bodeninsekten

Testinsekt: Tenebrio molitor-Larven (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigte z.B. bei einer Wirkstoffkonzentration von 20 ppm die Verbindung des Herstellungsbeispiels 3 eine Abtötung von 100 %.

Beispiel F

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigten z.B. bei einer Wirkstoffkonzentration von 20 ppm die Verbindungen der Herstellungsbeispiele 1,2, 3 und 8 eine Abtötung von 100%.

Beispiel G

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigte z.B. bei einer Wirkstoffkonzentration von 20 ppm die Verbindung des Herstellungsbeispiels 2 eine Abtötung von 100 %.

Das erfindungsgemäße Verfahren soll anhand der folgenden Herstellungsbeispiele erläutert werden:

Beispiel 1

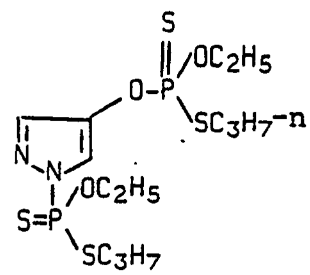

Zu einer Mischung aus 5 g (0,06 Mol) 4-Hydroxypyrazol, 24,8 g (0,18 Mol) Kaliumcarbonat und 150 ml Acetonitril gibt man bei 20°C 26,2 g (0,12 Mol) O-Ethyl-S-n-propyl-dithiophosphorsäurediesterchlorid. Anschließend wird das Reaktionsgemisch 5 Stunden bei 45°C nachgerührt. 300 ml Toluol werden zugegeben und die Lösung wird zweimal mit je 200 ml Wasser extrahiert. Die organische Phase wird über Natriumsulfat getrocknet, anschließend wird das Lösungsmittel im Vakuum abdestilliert.

Man erhält 22 g (82 %) O-Ethyl-S-n-propyl-O-[1-(n-propylthio-ethoxy-thionophosphoryl)-4-pyrazolyl]-dithiophosphorsäureester in Form eines gelben Oels mit dem Brechungsindex $n_D^{21}$ : 1,5518.

Analog diesem Beispiel können die folgenden Verbindungen der Formel I

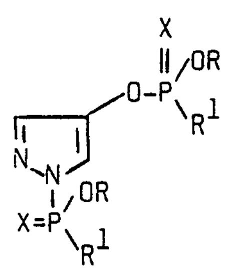

(I)

hergestellt werden.

| Beisp. Nr. | R | $R^1$ | X | Brechungsindex |
|---|---|---|---|---|
| 2 | $C_2H_5$ | $SC_3H_7$-n | O | $n_D^{21}$: 1,5159 |
| 3 | $C_2H_5$ | $OC_2H_5$ | S | $n_D^{21}$: 1,5050 |
| 4 | $C_2H_5$ | NH-$C_3H_7$-iso | S | $n_D^{21}$: 1,5178 |
| 5 | $CH_3$ | $OCH_3$ | S | $n_D^{21}$: 1,5291 |
| 6 | $C_2H_5$ | $C_2H_5$ | S | |
| 7 | $C_2H_5$ | 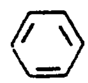 | S | |
| 8 | $C_3H_7$-n | $OC_2H_5$ | S | $n_D^{21}$: 1,4986 |
| 9 | $C_2H_5$ | $OC_3H_7$-iso | S | |

Das als Vorprodukt einzusetzende 4-Hydroxypyrazol kann z.B. wie folgt hergestellt werden :

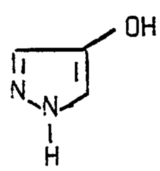

Eine Lösung von 19,6 g (0,2 Mol) 4-Methoxypyrazol (Archiv der Pharmazie 300, 704-708 (1967)) in 42 ml 63 prozentiger Bromwasserstoffsäure wird 5 Stunden unter Rückfluß gekocht. Dann destilliert man die überschüssige Bromwasserstoffsäure im Vakuum ab, löst den Rückstand in 80 ml Aceton und gibt 42 g (0,5 Mol) Natriumhydrogencarbonat zu. Das Gemisch wird 6 Stunden gerührt und dann filtriert. Das Filtrat wird im Vakuum eingedampft, der Rückstand in Acetonitril

gelöst, filtriert und erneut eingedampft. Zurück bleiben 18,3 g 4-Hydroxypyrazol, das noch ca. 10 - 15 % Natriumbromid enthält. Durch Umkristallisation aus halbkonz. Natriumchlorid-Lösung erhält man reines 4-Hydroxypyrazol mit dem Schmelzpunkt 127°C.

Patentansprüche

1. Neue O-(1-Phosphoryl-4-pyrazolyl)-phosphorsäureester der Formel I

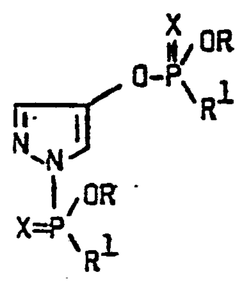

(I)

in welcher

R für gegebenenfalls substituiertes Alkyl steht,

$R^1$ für gegebenenfalls substituierte Reste aus der Reihe Alkyl, Alkoxy, Alkylthio, (Di)Alkylamino oder Aryl steht, und

X für Sauerstoff oder Schwefel steht.

2. Phosphorsäureester gemäß Anspruch 1, in welchen

R für geradkettiges oder verzweigtes Alkyl mit 1 bis 6 Kohlenstoffatomen steht,

$R^1$ für Alkyl, Alkoxy, Alkylthio und (Di)Alkylamino, jeweils mit geradkettigem oder verzweigtem Alkylrest und 1 bis 6 Kohlenstoffatomen, oder für Phenyl steht, und

X für Sauerstoff oder Schwefel steht.

3. Phosphorsäureester gemäß Anspruch 1, in welchen

R für Methyl, Ethyl, n-Propyl, i-Propyl-, n-Butyl, i-Butyl, sec.-Butyl und tert.-Butyl steht,

$R^1$ für Methyl, Ethyl, n-Propyl, i-Propyl, n-Butyl, i-Butyl, Methoxy, Ethoxy, n-Propoxy, i-Propoxy, n-Butoxy, i-Butoxy, Methylthio, Ethylthio, n-Propylthio, i-Propylthio, n-Butylthio, i-Butylthio, sec.-Butylthio, (Di)Methylamino, (Di)Ethylamino, (Di)n-Propylamino, (Di)i-Propylamino, (Di)n-Butylamino, (Di)iso-Butylamino, Methylethylamino, Methyl-n-propylamino, Methyl-i-propylamino, Methyl-n-butylamino, Methyl-i-butylamino, Ethyl-n-propylamino, Ethyl-i-propylamino, Ethyl-n-butylamino, Ethyl-i-butylamino, n-Propyl-i-propylamino, n-Propyl-n-butylamino, n-Propyl-i-butylamino, i-Propyl-n-butylamino, i-Propyl-i-butylamino, n-Butyl-i-butylamino und Phenyl steht, und

X für Sauerstoff oder Schwefel steht.

4. Phosphorsäureester gemäß Anspruch 1, in welchen

R für Methyl, Ethyl oder n-Propyl steht; und
$R^1$ für Ethyl, Methoxy, Ethoxy, i-Propoxy, n-Propylthio, i-Propylamino oder Phenyl steht; und
X für Sauerstoff oder Schwefel steht.

5. Phosphorsäureester gemäß Anspruch 1, in welchen

R für Ethyl steht; und
$R^1$ für Ethoxy oder n-Propylthio steht; und
X für Schwefel oder Sauerstoff steht.

6. Verfahren zur Herstellung des O-(1-Phosphoryl-4-pyrazolyl)-phosphorsäureester der Formel I

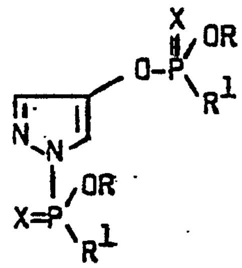

(I)

in welcher

**R** **für gegebenenfalls substituiertes Alkyl steht,**

**$R^1$** **für gegebenenfalls substituierte Reste aus der Reihe Alkyl, Alkoxy, Alkylthio, (Di)Alkylamino oder Aryl steht,** und

**X** **für Sauerstoff oder Schwefel steht,**

dadurch gekennzeichnet, daß man 4-Hydroxypyrazol der Formel II

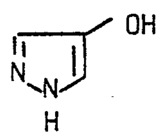

(II)

oder deren Alkalimetall-, Erdalkalimetall- oder Ammoniumsalze mit Halogeniden der Formel III

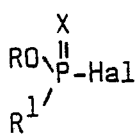

(III)

in welcher

R, $R^1$ und X die oben angegebene Bedeutungen haben, und

Hal für Halogen steht,

gegebenenfalls in Gegenwart eines Säureakzeptors und gegebenenfalls unter Verwendung eines Verdünnungsmittels umsetzt.

7. Schädlingsbekämpfungsmittel, gekennzeichnet durch einen Gehalt an mindestens einer Verbindung der Formel I.

8. Verwendung von Verbindungen der Formel I zur Bekämpfung von Schädlingen, insbesondere Insekten und Spinnentieren.

9. Verfahren zur Bekämpfung von Schädlingen, dadurch gekennzeichnet, daß man Verbindungen der Formel I auf die Schädlinge, vorzugsweise Insekten oder Spinnentiere oder ihren Lebensraum einwirken läßt.

10. Verfahren zur Herstellung von Schädlingsbekämpfungsmitteln, dadurch gekennzeichnet, daß man Verbindungen der Formel I mit Streckmitteln und/oder oberflächenaktiven Mitteln vermischt.